# EUROPEAN PATENT SPECIFICATION

(11) **EP 2 266 664 B1**
(45) Date of publication and mention of the grant of the patent: **16.10.2013**
(21) Application number: 10164658.6
(22) Date of filing: 01.06.2010
(51) Int. Cl.: A61N 5/10

(54) **System for dynamic strobe arc therapy**
System zur dynamischen Abtasrotationstherapie
Système pour traitement dynamique par arc stroboscopique

(30) Priority: 23.06.2009 US 219662 P; 18.08.2009 US 543437
(43) Date of publication of application: 29.12.2010
(73) Proprietor: Siemens Medical Solutions USA, Inc., Malvern, PA 19355-1406 (US)
(72) Inventor: Shukla, Himanshu P., Lafayette 94549 (US); Stahl, Johannes, Walnut Creek 94598 (US)
(74) Representative: Maier, Daniel Oliver

(56) References cited:
- US-A1- 2008 002 811
- US-A1- 2008 242 969

## Description

According to conventional radiation therapy, a beam of radiation is directed toward a tumor located within a patient. The radiation beam delivers a predetermined dose of therapeutic radiation to the tumor according to a treatment plan. The delivered radiation kills cells of the tumor by causing ionizations within the cells. A major concern is limiting the damage to healthy tissue surrounding the tumor.

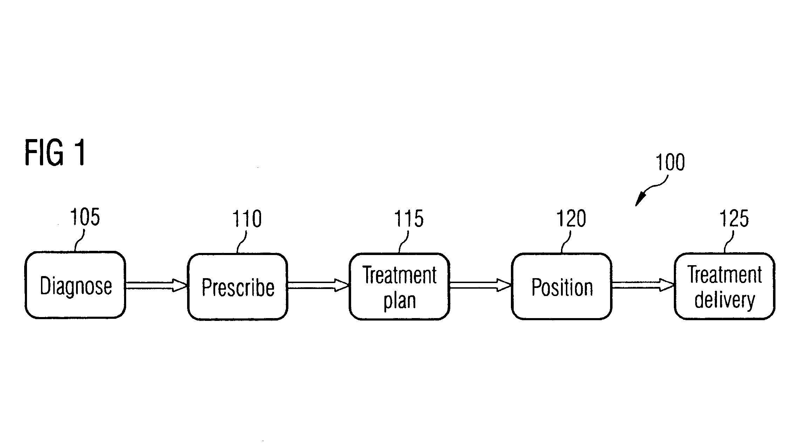

FIG. 1 illustrates a conventional patient treatment process that includes radiation therapy. According to some examples of process 100, image data of a patient is acquired, and a target volume and critical internal structures are identified based on the image data during diagnosis (105). A radiation dose is prescribed (110) for achieving desired results with respect to the target volume while minimizing damage to the critical structures. Next, a treatment plan for delivering the dose is determined (115).

The treatment plan is then delivered (125) to the patient during several sessions, or "fractions", spaced over some period of days. Prior to each fraction, the patient is positioned (120) as required by the treatment plan. Such positioning may involve the use of lasers, skin markers, etc.

Various methods or modes of radiation therapy delivery have been proposed and utilized. In prior systems, each radiation therapy delivery method is typically implemented using specific modes that require a control system optimized for one specific mode of radiation therapy delivery. Some modes of radiation therapy delivery include, for example, CT Guided IMRT (Intensity Modulated Radiation Therapy) , Volumetric Modulated Arc Therapy conventional IMRT, and Dynamic Modulated Arc Therapy. In a CT Guided IMRT system, such as a TomoTherapy® system provided TomoTherapy Incorporated, a linear accelerator is mounted in a ring-shaped gantry and moves in a 360 degree rotation around the patient. During delivery the beam is always on and it is partially blocked or unblocked by a binary MLC (multileaf collimator) that rapidly opens and closes the MLC leaves as the gantry rotates. In a Volumetric Modulated Arc Therapy system, such as provided by the RapidArc™ radiotherapy technology from Varian Medical Systems, an L-shaped gantry performs a 360 degree rotation around the patient. The beam is constantly on and the dose rate may be modulated. Also, the MLC leaves are also in constant motion, thus creating different shapes as the gantry rotates. In a conventional IMRT system, beams with modulated intensity are generated at a number of fixed positions or angles around the patient. These beams are then delivered with the gantry stationary at each fixed position. The beam intensity is modulated by either superimposing several shapes at a fixed position ("Step and Shoot IMRT") or by moving MLC leaves across the beam with varying speeds ("Sliding Window IMRT"). In a Dynamic Modulated ARC therapy system the gantry of the delivery system performs a contiguous rotational motion (360 degrees or less per arc). Throughout the rotational motion, the beam remains on at constant dose rate, and the MLC leaves constantly re-form to maintain a shape which is conformal with the shape of the tumor, as viewed from the respective angle.

During the planning stage (115) for the radiation therapy treatment, a decision is made regarding which delivery mode to use. The decision may be based on a number of factors, including for example the patient's diagnosis, delivery system constraints, time issues related to schedules and availability of the patient and/or radiation therapy systems, etc. After the specific mode of delivery is decided, the treatment plan is determined that is adapted to the specifically chosen delivery mode.

Systems have attempted to address various aspects of the foregoing. For example, a delivery mode may be chosen based on the location of a tumor and the surrounding healthy tissue, the capabilities of treatment systems available for treating the patient, etc. However, trade-offs or compromises may be made since a specific radiation therapy treatment mode is chosen and used. More flexible radiation therapy delivery methods are desired.

An example of a radiotherapy device is described in US 2008 00 28 11.

To address at least the foregoing, some embodiments provide a system, apparatus, and means to receive a radiation treatment plan for delivering at least a portion of a prescribed radiation dose to a target volume from different incidence directions around the target volume; and to deliver a portion of the prescribed radiation dose to the target volume by: a) moving a dynamic component along a path in order to dynamically change the incidence direction of the radiation beam to the target volume, wherein a plurality of path sections are disposed along the path, each of the path sections having a start position and a stop position, and b) delivering a portion of the prescribed radiation dose to the target volume at the path sections by applying radiation between the start position and the stop position of each path section, respectively.

Radiation is applied when the dynamic component arrives at and/or moves through the path section during its movement along the path. Each path section may be associated with one of a series of radiation dose portions.

Between at least some of successive path sections a gap may exist. The stop position of path section may differ from the start position of the successive path section. There may be a non-zero difference between the start position along the path of a path section and the stop position along the path of the previous path section. A gap may in particular exist between each pair of successive path sections, i.e. after the stop position of each path section a non-vanishing gap may exist along which the dynamic component must move until the start position of the next path section is reached. When the dynamic component moves along a gap between two successive path sections when moving along the path, radiation is not applied.

At least some of the path sections, however, have a start position that differs from the stop position. The difference is non-zero. For such path sections that have a non-vanishing extension, radiation is applied continuously as the dynamic component moves along the path section. All path sections may have a non-vanishing extension.

The path section length of some or all path sections may be determined such that the radiation dose distribution applied at these path sections does not deviate more than 30%, in particular not more than 20% or 10% or 5% or 3% compared to radiation dose distribution with an equivalent fixed beam delivery (same system parameter setting except that the dynamic component is not moving and fixed at the center position in the path section). This may apply for some or all path sections with non-vanishing extension. This allows simplified dose calculation and quality checks.

During a radiotherapy session, the dynamic component moves along the path. When the dynamic component moves to a start position and reaches the start position of a predefined path section, the system starts to apply radiation. The dynamic component does not have to stop at the start position. The dynamic component keeps on moving along path section until the stop position of this path section has reached. When stop position has been reached, the system stops to apply radiation. The dynamic component may nevertheless continue its movement along the path. With this radiation application scheme, it is possible to save time as they dynamic component does not have to stop in order to deliver radiation.

The dynamic component is configured to set and/or to change the incidence direction to the target volume. Changing the incidence direction means directing the central beam ray of the radiation beam to a different location in the target volume and/or with a different incidence angle to the target volume.

A gantry that rotates the therapeutic radiation source around the target volume may thus be such a dynamic component. The path the gantry moves along is an arc. The plurality of path sections may thus be called "arclets", each arclet having a defined start angle as a start position and a defined stop angle as a stop position.

A treatment table configured to change the position of the target volume may also be a dynamic component. The path may be formed by continuously moving the target volume with the treatment table and thus changing the incidence direction of the radiation beam. The treatment table may for example translate the target volume along a path or rotate the target volume of perform even more complex movements by combining rotational and translational movement.

The path along which the dynamic component moves may be configured such that the incidence direction for the different treatment beams or the different path sections do not overlap. This means that radiation is always applied from a different incidence direction. The path may be configured such the incidence direction of radiation may be different for each point along the path. For example, when the dynamic component is a gantry that performs a gantry rotation around the target volume, the whole path may be an arc of 360° or less. The gantry may only perform one single rotation or less. Even when the gantry moves along an arc of more than 360°, the arclets where radiation is applied from may be arranged such that the arclets do not overlap.

The gaps between the path sections may be used to change or adapt the radiotherapy system setting for the next path section. For example, the beam energy, the dose rate, the beam shape and/or the gantry speed may be adjusted or changed for the radiation delivery in the following path section. The time the dynamic component needs to reach the next arclet may be used to change settings of system components, e.g. to set a new beam energy, a new beam shape and/or a new dose rate. Then, at the beginning of the next path section, the gantry may not need to stop but may still continue its movement, and radiation may be turned on with the new system settings. This section-wise application scheme allows speeding up application of the full prescribed radiation dose.

Compared to systems where the gantry moves continuously around 360° and applies continuously radiation, the new scheme with gaps between path sections has several advantages. Because of the gaps between path sections during which system settings may be changed, less system constraints have to be considered during therapy planning as in systems where radiation is continuously applied when the gantry is moving. As during the gaps no radiation is applied, the system may change parameter setting with a high degree of freedom at those gaps: The radiation applied to the target volume is not influenced by the way how parameter settings are changed and set to new values in the gaps. The system constraints imposed by the system have a much minor impact on the dose delivery, as changes are made in radiation-silent gaps. The system may even change parameters during the gap which are non-contiguous or which cannot be changed at all while radiation is being delivered.

Furthermore, dose calculations may be simplified compared to systems where the gantry continuously applies radiation around an arc without interruption.

The system does not continuously apply radiation, but only at the path sections. This scheme allows driving the system with much higher dose rates as compared to systems where the gantry moves around 360° and continuously applies radiation. Latter systems must turn down the dose rate as the beam in continuously on, otherwise it would be likely to apply too much total dose. In order to allow similar high dose rates, systems with continuous radiation application would require very fast responsive (and thus expensive) components.

As the radiation is not applied continuously, but only at defined path sections, the radiation application scheme may be called "burst-mode" scheme or "strobe-mode" scheme. As radiation may be applied during movement of the dynamic component, the scheme may be called dynamic strobe mode scheme. Radiation may be applied discontinuously in bursts, and during bursts the dose rate may be much higher than in conventional continuous application schemes. The overall time the beam is turned on is reduced.

The overall time where radiation is applied may add up to less than 60%, in particular less than 50% and in particular less than 40%, 30% or 20% of the travel time where dynamic component moves along the whole path. This may be achieved by determining the path sections, the movement characteristics of the dynamic component and/or the dose rate for the application or radiation at the path sections accordingly. With respect to the length of path sections where radiation is applied, the length of all the path sections where radiation is applied may add up to less than 60%, in particular less than 50% and in particular less than 40%, 30% or 20% of the whole path length the dynamic component moves along. Such constraints have the benefit that - if radiation is applied - the dose rate is high and that the time radiation is ON is reduced. The dose rate can be high, in particular more than 1000MU/min, 2000MU/min, 3000MU/min or 4000MU/min. These dose rate constraints may apply to some or all path sections. This burst-like application with short bursts of high intensity has advantages, in particular because adverse effects of moving targets are reduced. In particular if some system parameter settings are kept constant during the application of radiation (e.g. due to the implementation of the method), this applies only to the short interval of high intensity bursts. The rest of the time may be used to adjust system parameter settings. This allows more pronounced changes parameters of system parameter settings during the gaps without the need to modify the complete system capabilities.

The length of the path sections may be determined, in particular by the delivery system, such that a given radiation dose portion may be delivered at the respective path section. The determination of the length may take into account the time the dynamic component needs to move along the path section and/or the dose rate the radiation dose portion is applied with. The system may optimize some or all of these parameters under the constraint that the time that is needed to apply the prescribed radiation is reduced or even minimized.

The length of the path sections may be determined, in particular by the delivery system, such that the respective path length is less than a prescribed path section length. A prescribed path section length may be used for example by a treatment planning system to determine the treatment plan, i.e. the radiation dose portions and the assigned incidence directions. Using one or more prescribed path section lengths ensure that an applied radiation dose corresponds to the desired radiation dose, even if the ultimate determination of path sections lengths is not done in full detail by the treatment planning system.

At least some of the path sections, however, have a start position that differs from the stop position. The difference is non-zero. For such path sections that have a non-vanishing extension, radiation is applied continuously as the dynamic component moves along the path section. It is also possible that all path sections have a non-vanishing extension.

The dynamic component may move at constant speed when applying radiation between the start position and the stop position of a path section. Such an embodiment provides simplified control of the system. The speed of the dynamic component may be adjusted when the dynamic component moves along the gaps between successive path sections. However, the speed of the dynamic component may also be modulated during path section where radiation is applied, for example to react to disturbances or other external events.

The radiation may be applied constant dose rate. However, it is also possible to vary the dose rate as the dynamic component moves along one of the path sections.

Some of the radiotherapy system setting may be used and set as discrete parameters, i.e. parameters that are changed at the gaps and that remain constant when the dynamic component moves at least through path sections with non-vanishing extension. Examples for discrete parameters are a beam shape, as is may be defined by a collimator setting, a beam energy, a speed of the dynamic component and/or a dose rate, beam type (e.g. photon vs. electron). Having a system setting parameter as a discrete parameter simplifies control of the system at the expense of less flexibility regarding radiation application. Especially when moving through a gap, the speed of the dynamic component may be adjusted such that the slowest discrete parameter may be changed to its new value. This means that out of the set of all discrete parameters that are to be changed, it is sufficient to just determine the one discrete parameter which needs the most time to be changed. When the speed of the dynamic component is adapted to this parameter, it is automatically guaranteed that all other discrete parameters may be changed until the next path section begins.

Some settings for the radiotherapy system may be used and set as continuous parameters, i.e. parameters that are changed even when the dynamic component moves through path sections. Examples for continuous parameters are a beam shape, as is may be defined by a collimator setting, a beam energy, a speed of the dynamic component and/or a dose rate. Having a system setting parameter as a continuous parameter increases the flexibility regarding radiation application; it makes, however, the control of the system more complex as constraints imposed by the system have to be considered more carefully as these continuous parameters change during radiation application.

Each path section may be characterized by a plurality of beam parameters such as a defined beam shape, a defined beam energy and/or a defined radiation dose that is about to be applied. These parameters may change from path section to path section and may be set to a new value when the dynamic component moves along the path in the gaps between two successive path sections. These parameters may be kept constant as the dynamic component moves along one of the path sections, at least for path sections that have a non-vanishing extension. If desired, the correct setting of the beam parameter may be verified before the radiation is applied again at the subsequent path section.

During application of the radiation at one, some or all path sections, the system may maintain its configuration for many system parameter settings except for the position of the dynamic component. No other beam parameters or system parameters may need to change during the motion of the dynamic component from the start position of path section to the stop position and during application of radiation. Beam characteristics as the dose rate, beam shape and/or beam energy may be kept at steady state during the path section. Such an embodiment allows a simplified control of the system.

For example, the settings of a beam shaping device as a collimator may be kept constant when applying radiation between the start position and the stop position of some or each path sections. The collimator leafs may not move and/or the collimator's rotational position may be kept constant. Such an embodiment also allows a simplified control of the system. The settings of a beam shaping device as a collimator may be changed to a new shape when the dynamic component moves along a gap between two successive path sections. For example, collimator leaves may be controlled to assume a different position or the rotational angle of a collimator may be controlled at the gaps.

In some embodiments, a shape and/or dose of each of the radiation dose portions or for each of the path sections may be optimized by the treatment plan. A number of different methods, algorithms, and techniques may be used during the treatment planning stage to create the treatment plan. In some embodiments, the treatment plan may be created using a Direct Aperture Optimization algorithm.

In some aspects herein, each path section may also defined by beam parameter, such as a beam shape, a beam dose, and/or a beam energy. To ensure adherence to a desired treatment plan, the beam dose for each path section is delivered between the start position and the stop position. In some embodiments, the beam dose may be distributed between the start position and the stop position at a maximum dose rate. The maximum dose rate may be centered at about a mid-point between the start position and the stop position for some or all path sections. In some other embodiments, the beam dose may be distributed substantially equally between the start position and the stop position for some or all path sections.

In some embodiments one or some of the path sections may have a start position that is equal to the stop position of the path section. Such path sections have a vanishing extension and are point-like. At these point-like path sections, the dynamic component may stop its movement for applying radiation. In the case of a revolving gantry, the start angle and the stop angle for at least one of the arclets may coincide with each other. In these and other instances, the systems of the present disclosure may provide a flexible radiation therapy delivery mode that can accommodate other radiation therapy delivery modes, at least during one segment.

When the dynamic component is stopped at one of these point-like path sections, the beam shaping device (or other system parameters as dose rate or beam energy, for example) may be changed several times and more than one radiation dose portion may be applied in successive order with different system parameter setting at this point-like path section. Additionally, the path sections of the radiation therapy embodiments presented herein may be organized to reduce a time needed to deliver at least a portion of the prescribed radiation dose to the target volume. The path sections may be organized to address other issues or concerns in some embodiments.

The various aspects and embodiments of the invention as described herein may be implemented in different combinations.

Some aspects of the invention provide a system, method, apparatus, and means for radiotherapy planning comprising: determining at least a portion of a prescribed radiation dose to a target volume to be applied from different incidence directions to the target volume; and determining a plurality of path sections, the path sections being distributed along a path that characterizes a movement of a dynamic component, wherein the dynamic component is adapted to change the incidence direction of the radiation to the target volume, and wherein each of the path sections has a start position and a stop position; and determining a plurality of radiation dose portions that are assigned to the path sections, respectively, to be applied between the start position and the stop position of the path sections, such that the total overall dose that is to be applied corresponds to a desired dose distribution.

The plurality of radiation dose portions may be determined under the constraint of a gap arranged between at least some of successive path sections, respectively.

A beam parameter, in particular a beam shape, a beam energy and/or a dose rate, may be determined for each path section, wherein the beam parameter is determined under the constraint that the beam parameter is kept constant between the start position and the stop position for at least some of the path sections.

At least one of the path sections may be determined such that they have a start position equal to the stop position.

Single points along the path may be associated with each of the radiation dose portions and may be converted to path sections having a start position and a stop position. The path sections may be derived from single points distributed along the path that are converted into the path sections.

The plurality of radiation dose portions may be determined under the constraint that a maximum dose rate is delivered between the start position and the stop angle position for at least some of the path sections. The plurality of radiation dose portions may be determined under the constraint that a beam dose is distributed substantially equally between the start position and the stop position for at least some of the path sections.

The plurality of path sections and/or the beam parameters associated with the path sections may be determined under the constraint to reduce a time to provide the delivering of the portion of the prescribed radiation dose.

Constraints as described above for the radiotherapy system may be applied also during the planning phase for determining the plurality of path sections distributed along the path and/or the respective radiation dose portions that are to be applied and/or their characteristics.

A radiotherapy planning system may comprise a computer system with an input device for inputting treatment planning information to the computer and for allowing interaction with a user and with an output device for outputting processed treatment planning information. The computer system may further comprise a processor being adapted to implement a radiotherapy planning method as described.

Embodiments are not limited to those described herein, as those in the art can readily adapt the descriptions to create other embodiments and applications. The invention is defined in the claims.

The construction and usage of embodiments will become readily apparent from consideration of the following specification as illustrated in the accompanying drawings, in which like reference numerals designate like parts, and wherein:
FIG. 1 is a diagram of a radiation therapy process, in accordance with some embodiments herein;
FIG. 2 is a flow diagram of a dynamic strobe modulation ARC therapy process, according to some embodiments;
FIG. 3 is a flow diagram of a dynamic strobe modulation ARC therapy process, according to some embodiments;
FIG. 4 is a perspective view of a radiation therapy system, according to some embodiments;
FIG. 5 is a block diagram of a radiation therapy system, according to some embodiments; and
FIG. 6 comprises a flow diagram illustrating a dynamic strobe modulation ARC therapy processes, according to some embodiments herein.
FIG. 7 shows a portion of an arc a gantry of a radiotherapy device moves along with path sections where radiation is applied,
FIG. 8 shows a portion of an arc a gantry of a radiotherapy device moves along with path sections where radiation is applied with one point-like path section,
FIG. 9 shows a diagram with an illustration of the time course of radiation with path sections where radiation is applied,
FIG. 10 shows a diagram with an illustration of the time course of radiation with path sections where radiation is applied with one point-like path section where movement of the dynamic component is stopped.

The following description is provided to enable any person in the art to make and use the embodiments described herein and sets forth the best mode contemplated therefore. Various modifications, however, will remain readily apparent to those in the art.

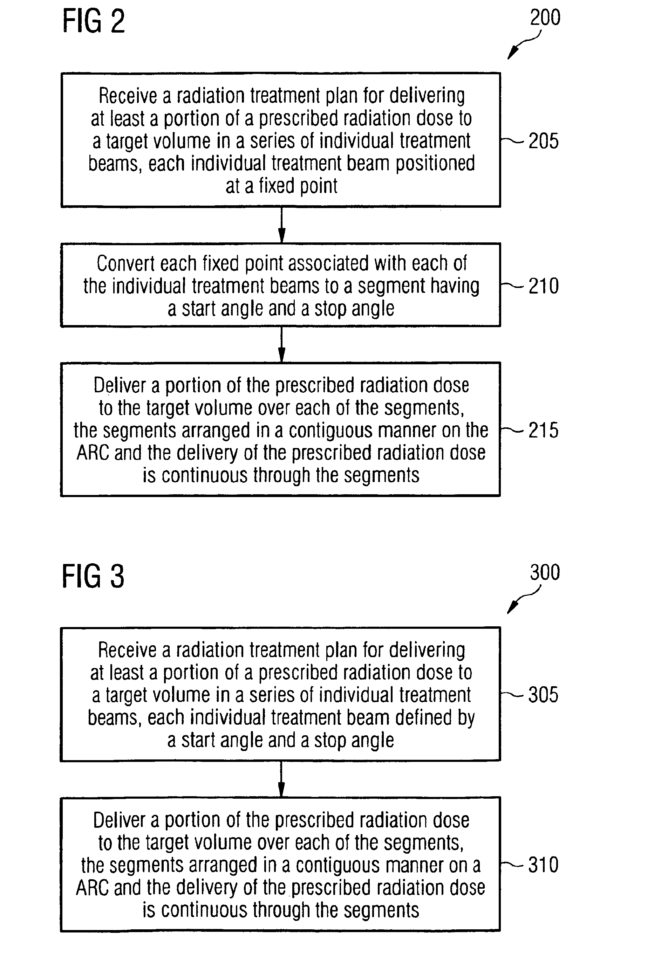

FIG. 2 is an illustration of a dynamic strobe modulation ARC therapy process 200, according to some embodiments. The illustrated process 200 may be implemented by any suitable hardware and/or software elements. Some embodiments may include hardware elements, some embodiments may include software elements, and other embodiments may include both software and hardware elements in the implementation of the illustrated processes, systems, and devices herein. The process of FIG. 2 is not limited to the order shown therein. Rather, embodiments of the process may be performed in any order that is practicable.

At operation 205, a radiation treatment plan for delivering at least a portion of a prescribed radiation dose to a target volume is received by a radiation therapy system. In some embodiments, the radiation treatment plan is provided to the radiation therapy treatment delivery system embodied in a computer or processor readable medium such as a file or series of files embodied in a memory storage unit. The memory storage unit may be implemented as an optical disk, a CD-ROM, RAM, a flash ROM, or any type of memory storage unit now known or that becomes known in the future.

In some embodiments, the radiation treatment plan received or otherwise provided at operation 205 may be created using the Direct Aperture Optimization (DAO) algorithm. In accordance with the DAO algorithm, the DAO treatment planning algorithm may create a series of individual radiation dose portions that are placed in a circular configuration around the patient. The location of each radiation dose portion is specified by a fixed point. The angular position of each radiation dose portion is called the Optimization Point (OP). The shape and/or dose of each radiation dose portion is optimized by the DAO algorithm to achieve an optimum dose distribution in the patient based on the planning criteria.

In some embodiments, planning algorithms other than the DAO algorithm may be used to create the treatment plan. For example, a treatment plan created for Conformal Arc Therapy (not based on the DAO algorithm) may be used to create the treatment plan received at operation 205. That is, the treatment planning algorithm, system or methodology used herein may include other treatment planning algorithms in addition to, as a substitute for, a modification of, and an alternative to the DAO algorithm.

As an example, a treatment planning system (not shown) may include a DAO planning system that generates, for example, 36 OP's spaced at 10 degree intervals between each OP around the patient. Again, the shape and the dose of each shape is optimized by the DAO algorithm to achieve an optimum dose distribution in the patient based on the planning criteria.

At operation 210, each OP associated with each of the radiation dose portions is converted to a segment having a start angle and a stop angle. Each segment extends over a range starting with the start angle and terminating with the stop angle. The delivery system herein converts the OP's into segments with defined start and stop angle in order to control the deviation from the original, point-based optimization provided in the treatment plan received at 205. Each segment represents the range over which a prescribed radiation dose associated with an OP is to be delivered, instead of being delivered at the fixed point specified by each OP.

The converting of operation 210 to "spread" the radiation dose portion around the OP between the segment start angle and stop angle may be anticipated by the planning system that generates and provides the treatment plan at 205. The "spreading" of the radiation dose portion dose may thus be considered and included in the dose calculation to further reduce any small inaccuracies that may result from the converting of operation 210.

Continuing the example introduced above, each of the 36 OP's may be converted into a segment having a defined start angle and a stop angle. Since the treatment plan included 36 OP's, a "final" treatment plan including the segments obtained as a result of converting the OP's to segments includes a sequence of segment for delivering the prescribed radiation dose(s).

At operation 215, a portion of the prescribed radiation dose is delivered over each of the segments. It is noted that the segments are arranged in a contiguous manner in the arc around the patient and the delivery of the prescribed radiation dose is continuous through the segments.

Accordingly, a radiation therapy system configured to deliver radiation therapy in accordance with process 200 may include a gantry that moves in a continuous motion around the patient, and delivers radiation dose portions at the OP's without stopping. The dose may be delivered in short strobes (or bursts) of high intensity radiation (e.g., the maximum available dose rate) such that the deviation from the original plan caused by the continuous gantry motion is reduced or minimized.

In a traditional step-and-shoot IMRT methodology, the gantry moves from incidence angle to incidence angle, stops at each position, delivers the radition dose at that position, and then moves to the next fixed position to deliver the next radiation dose, and so on. As compared to the process of FIG. 2, the traditional step-and-shoot IMRT methodology is very time-consuming.

In some embodiments, a 'final' treatment plan in accordance herewith (e.g., obtained at 210) may include a sequence of delivery segments, within which discrete points are described primarily by the parameters of: a beam shape, a beam dose, a beam energy, and gantry angles defining a range or span of the segment (i.e., an upper and lower limit) within which the radiation dose shall be delivered.

In some embodiments, a planning system, module, or mechanism may optimize or otherwise provide a segment for delivery of individual radiation dose portions. FIG. 3 is an illustration of a dynamic strobe modulation ARC therapy process 300, according to some embodiments. The illustrated process 300 may be implemented by any suitable hardware and/or software elements. Some embodiments may include hardware elements, some embodiments may include software elements, and other embodiments may include both software and hardware elements in the implementation of the illustrated processes, systems, and devices herein.

At operation 305, a radiation treatment plan for delivering at least a portion of a prescribed radiation dose to a target volume is received by a radiation therapy system. In some embodiments, the radiation treatment plan is provided to the radiation therapy treatment delivery system embodied in a computer or processor readable medium such as a file or series of files embodied in a memory storage unit. The memory storage unit may be implemented as an optical disk, a CD-ROM, RAM, a flash ROM, or any type of memory storage unit now known or that becomes known in the future.

In some embodiments, the radiation treatment plan received or otherwise provided at operation 305 may provide a series of segments of individual radiation dose portions defined, at least in part, by a start angle and a stop for each segment. The segments may be optimized by a treatment planning system, module, or mechanism based on the planning criteria using one or more radiation therapy planning algorithms, systems, techniques, and methodologies.

At operation 310, a portion of the prescribed radiation dose is delivered to the target volume over each of the segments as defined by the start and stop angles of the segments. It is noted that the segments are arranged in a contiguous manner in an arc around the patient and the delivery of the prescribed radiation dose is continuous through the segments.

Accordingly, a radiation therapy system configured to deliver radiation therapy in accordance with process 300 may include a gantry that moves in a continuous motion around the patient, in particular around a circular arc, and delivers the radiation dose portions at the OP's without stopping, and the dose may be delivered in short strobes (or bursts) of high intensity radiation.

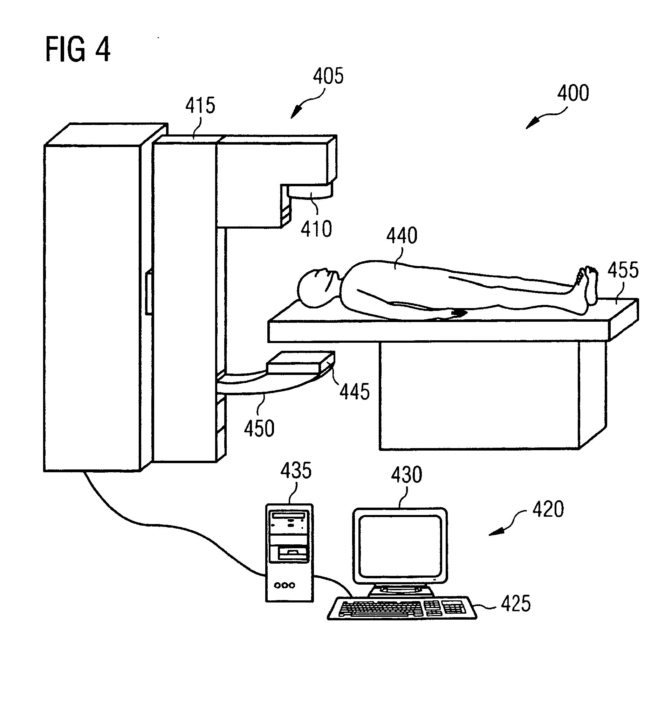

Embodiments of the processes herein will be further described in detail below. Such embodiments will be generally described in conjunction with system 400, a perspective view of which is provided in FIG. 4. Of course, systems other than system 400 particularly depicted in FIG. 4 may be used to implement embodiments described herein.

System 400 includes linear accelerator 405, operator console 420, patient 440, imaging device 445, and table 455. System 400 may be used to generate radiation for imaging and/or for radiation therapy. In this regard, patient 440 is positioned to receive a radiation dose according to a radiation treatment plan.

Linear accelerator 405 may deliver a radiation beam from treatment head 410 toward a volume of patient 440 that is located at an isocenter of accelerator 405. According to some embodiments, the radiation beam may comprise photon or electron radiation having energies in the megavoltage range. Treatment head 410 includes a beam-emitting device (not shown) for emitting a radiation beam and a beam-shielding device or collimator (not shown) for shaping the beam and for shielding sensitive surfaces from the beam. Treatment head 410 may also include an accessory tray to receive and securely hold attachments used during the course of treatment planning and treatment (such as, for example, reticles, wedges, or the like).

Imaging device 445 may comprise any system to acquire two-dimensional images based on photon radiation (i.e., X-rays) and/or electron radiation received from treatment head 410. Accordingly, imaging device 445 may be suitable for acquiring image data based on megavoltage radiation. Imaging device 445 may be used to acquire images for diagnosis, for verification and recordation of a patient position, for verification and recordation of internal structure positions, and/or for other purposes. In some instances, cone-beam reconstruction techniques may be used to construct three-dimensional images from two-dimensional images acquired by imaging device 445.

In some embodiments, imaging device 445 may be a flat-panel imaging device using a scintillator layer and solid-state amorphous silicon photodiodes deployed in a two-dimensional array. In other embodiments, imaging device 445 converts X-rays to electrical charge without requiring a scintillator layer. In such imaging devices, X-rays are absorbed directly by an array of amorphous selenium photoconductors. The photoconductors convert the X-rays directly to stored electrical charge that comprises an acquired image of a radiation field. Imaging device 445 may also comprise a CCD or tube-based camera. Such an imaging device may include a light-proof housing within which are disposed a scintillator, a mirror, and a camera.

Gantry 415 is rotatable around an axis before, during and after emission of the radiation beam. Rotation of gantry 415 may cause treatment head 410 and imaging device 445 to rotate around the isocenter such that the isocenter remains located between treatment head 410 and imaging device 445 during the rotation. Imaging device 445 may be attached to gantry 415 in any manner, including via extendible and retractable housing 450.

Table 455 supports patient 440 during radiation therapy. Table 455 may be adjustable to ensure, along with rotation of gantry 415, that a volume of interest is positioned between treatment head 405 and imaging device 445. Table 455 may be adjusted along any number of axes to accommodate any number of offsets in a position of patient 440.

Operator console 420 includes input device 425 for receiving instructions from an operator and output device 430, which may be a monitor for presenting operational parameters of linear accelerator 405 and/or interfaces for receiving instructions. Such instructions may include a selection from among a plurality of available radiation therapy processes. Output device 430 may also present images acquired by imaging device 445 to verify patient positioning prior to treatment delivery. Input device 425 and output device 430 are coupled to processor 435.

Processor 435 executes program code according to some embodiments. The program code may be executable to control system 400 to operate as described herein. The program code may be stored in storage media of identical or different types, including but not limited to a fixed disk, an optical disk, flash memory, a CD-ROM, a DVD-ROM, a disk, a magnetic tape, and any other storage medium now known or that becomes known.

Operator console 420 may be located apart from linear accelerator 405, such as in a different room, in order to protect its operator from radiation. For example, accelerator 405 may be located in a heavily shielded room, such as a concrete vault, which shields the operator from radiation generated by accelerator 405.

FIG. 4 may include less or more elements than those shown. In addition, embodiments are not limited to the system and devices shown in FIG. 4.

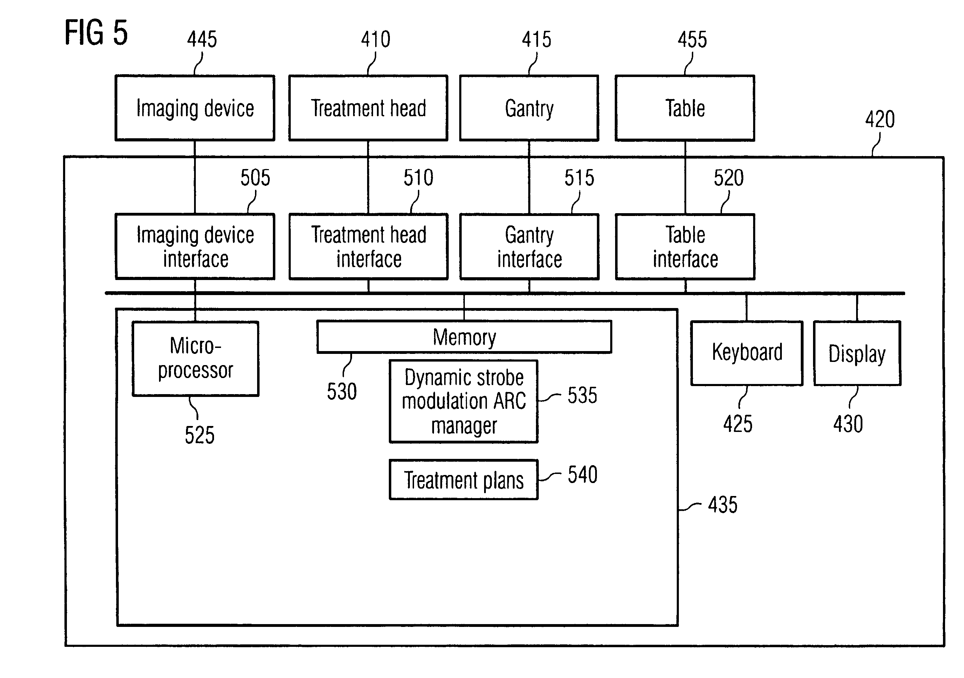

FIG. 5 is a block diagram of elements of system 400 according to some embodiments. As shown, operator station 420 includes several elements for interfacing with other elements of system 400. Specifically, operator station 420 includes imaging device interface 505, treatment head interface 510, gantry interface 515, and table interface 520.

Interfaces 505 through 520 may comprise dedicated hardware and/or software interfaces, and one or more of interfaces 505 through 520 may reside in processor 435. One or more of interfaces 505 through 520 may be implemented by a single interface. For example, interface 505 may be implemented by a single Ethernet interface and interfaces 510 through 520 may be implemented by a single proprietary interface for interfacing with table 455, treatment head 410, and gantry 415.

Processor 435 includes microprocessor 525 and memory 530. Microprocessor 525 may execute processor-executable program code stored in memory 530 to provide some or all of the functionality described herein. In this regard, memory 530 stores processor-executable process steps of dynamic strobe modulated ARC therapy manager 535.

Dynamic strobe modulated ARC therapy manager 535 may comprise processor-executable program code to implement process steps 200 and 300. Dynamic strobe modulated ARC therapy manager 535 may also comprise program code to generate and/or modify a treatment plan according to some embodiments.

Memory 530 may also store treatment plans 540 in accordance with any currently- or hereafter-known format. Treatment plans 540 may comprise scripts that are automatically executable by linear accelerator 405 and treatment table 455 to provide radiation therapy fractions. Treatment plans 540 may include one or more treatment plans in which a patient position, a beam plan, and/or a prescribed dose that have been optimized according to some embodiments herein, including but not limited to a DAO algorithm.

Usage of each of modules 535 and 540 will be discussed below, and may comprise any suitable program code to perform the functions attributed thereto. Modules 535 and 540 may comprise any suitable software format, including but not limited to a dynamic link library, a plug-in, an operating system extension, a standalone application, etc. Dynamic strobe modulated ARC therapy manager 535 may comprise module 540 or any other module such as a treatment planning module (not shown), according to some embodiments.

As part of the delivery scheme presented herein, the delivery system of FIGS. 4 and 5 may deliver the radiation beam portions herein, in general, according to the following rules or guidelines.

The delivery system will guarantee that each segment is delivered with the correct beam shape, wherein the beam shape will be verified prior to each segment delivery.

The delivery system will deliver the prescribed dose while passing between the gantry start angle and the gantry stop angle.

The delivery system will distribute the prescribed dose between the start angle and the stop angle in, for example, the following ways:

The delivery system will deliver the prescribed dose at maximum dose rate, centered about a mid-point of a prescribed path section window with a predefined path section length between the start angle and the stop angle. This distribution is referred to herein as a "Performance Mode".

The delivery system will adjust the prescribed dose rate such that the dose is distributed equally between the start angle and the stop angle of a prescribed path section window with a predefined path section length. This distribution is referred to herein as "Precision Mode".

The gantry speed of the delivery system may be adjusted for each path section separately such that an overall, aggregate delivery time for all segments is minimized. This may be accomplished by predicting the amount of time required for delivering the entire path section, including beam shaping and all other activities, and adjusting the gantry speed accordingly.

If the gantry start angle and the stop angle coincide, the gantry will stop at the prescribed position and will resume movement after the beam has been delivered.

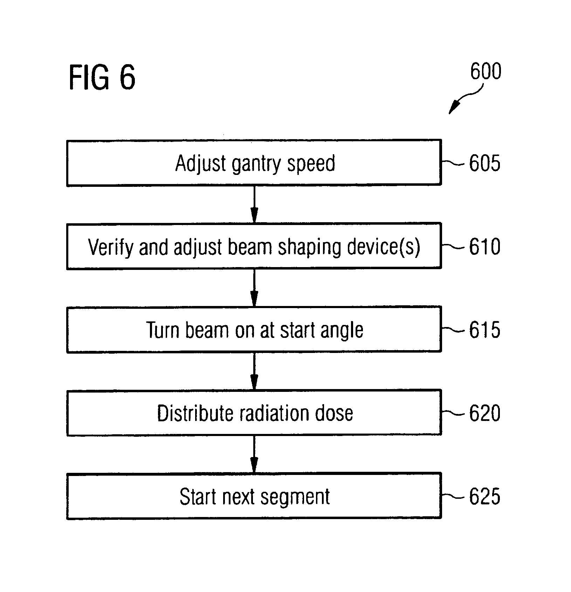

In some embodiments, and in agreement with the general rules or guidelines discussed above, a flow diagram illustrating a dynamic strobe modulation ARC therapy delivery process 600 is depicted in FIG.6, according to some embodiments herein. FIG. 6 is, in some aspects, a flow diagram of operation 215 of process 200 and of operation 315 of process 300. More specifically, process 600 is intended to represent a delivering of a radiation dose, according to some embodiments. Process 600, as well as all other process steps described herein, may be embodied in whole or in part by hardware of and/or software executed by elements including but not limited to those of system 400. Software (i.e., program code) embodying one or more of the process steps may be stored by any medium, including a fixed disk, flash memory, an optical disk, a CD-ROM, a DVD-ROM, a magnetic tape, or any other storage medium. Some or all of such software may also be stored in one or more devices. The process of FIG. 6 is not limited to the order shown therein. Rather, embodiments of the process may be performed in any order that is practicable. As stated earlier, unless stated otherwise, any methods and processes disclosed herein may be performed in any order that is practicable. Notably, some embodiments may employ one or more portions of the process arranged in different configurations without one or more other portions of the process.

Process 600 may be understood in the context of processes 200 and 300. With regard to process 200, the description will therefore assume that, prior to process 600, operations 205 and 210 have been executed and that a suitable 'final' treatment plan is provided for delivery by the operation 210. With regard to process 300, the description will assumes that a suitable 'final' treatment plan is provided for delivery by the operation 305.

Operation 605 includes adjusting the speed of the gantry (415) of the delivery system (400). In some embodiments, operation 605 may entail adjusting the gantry speed to a precalculated value by the delivery system. The speed of the gantry may be calculated by using a prediction of a duration of one segment, more than one segment, or all of the segments in order to minimize a treatment time while also satisfying the start angle and stop angle constraints of the segments. The duration time of the segments may include time for beam shaping, beam delivery, and other factors.

At operation 610, the beam shaping device(s) may be commanded to move to the prescribed shape of the beam. Operation 610, like operation 605, will occur prior to the delivery of a radiation dose protion during a subject segment. The shape of the beam shaping device(s) may be verified and adjusted in a variety of different manners including, in some embodiments, in real-time as the gantry transitions from one segment to a next segment.

Operation 615 includes a process wherein the beam is turned on and the target volume is irradiated. Operation 615 is initiated when the moving gantry reaches the prescribed start angle. Continuing with process 600 at operation 620, the radiation dose portion is distributed between the start angle and the stop angle. The distribution of the dose may be accomplished in a number of different manners, including the Precision Mode and the Performance Mode discussed herein. For example, in Precision Mode the dose rate of the beam will be set such that under constant gantry speed, the end of the dose delivery will coincide with the gantry stop angle of a prescribed path section window. Furthermore, when the prescribed dose has been delivered, the segment ends and the next segment starts, as illustrated at operation 625.

In some embodiments, the beam distribution of the dose may be implemented using a number of different techniques, mechanisms, and methodologies that may vary to deliver the prescribed dose. As such, for example, the speed of the gantry may be dynamically adjusted at or near the start angle, at or near the stop angle, and/or at or near a mid-point (or other multiple points) between the start angle and the stop angle. The speed of the gantry, and other aspects of the delivery system, including but not limited to the size and energy of the treatment beam or the dose rate, may be dynamically adjusted based on different criteria related to a treatment plan and patient. In some embodiments, the rate of the beam distribution of the dose between the start angle and the stop angle may, either alone or in combination with other beam dose distribution optimization aspects, be maximized by using a beam without a flattening filter.

FIG. 6 provides a general flow of beam delivery in accordance with embodiments and aspects herein. However, some embodiments may be configured, adapted, or otherwise have functionality to handle exceptions or special situations that may occur in conjunction with FIG. 6. For example, a radiation delivery system in accordance with embodiments herein may handle the following exceptions:

In an instance the beam shaping device does not set to the desired shape by a required time according to the treatment plan, the system will slow down the gantry and eventually stop the gantry (if need be) such that the dose can be delivered within the prescribed start angle and stop angle for the respective segment.

In an instance the beam shaping device fails to set the desired shape altogether, the treatment will be interrupted (i.e., aborted).

It is noted that the Dynamic Strobe Modulated ARC Therapy delivery model disclosed herein may encompass other modes of radiation therapy. For example, conventional IMRT may be expressed as a special case of the dynamic strobe delivery wherein the gantry start angle and stop angle of any given segment are equal to each. Accordingly, the prescribed radiation dose will be delivered with a stopped gantry, rather than being delivered with a continuously moving gantry as is the general case in accordance with the Dynamic Strobe Modulated ARC Therapy delivery model disclosed herein.

In some embodiments, hybrid delivery plans may be developed and executed that may include at least one 'standard' IMRT segment, as well as at least one 'dynamic strobe' segments as disclosed herein. The combination or hybrid delivery plans and modes including both 'standard' IMRT (or other modes) segments and at least one 'dynamic strobe' segments in any combination, to meet the treatment objectives. In this manner, a hybrid delivery plan may be developed and administered that includes the advantages of both methods (faster delivery with higher precision where required). For example, a hybrid treatment scheme may provide the accuracy of fixed IMRT in the most critical areas, while preserving the speed advantage of the 'dynamic strobe' ARC delivery wherever accuracy has less impact on the estimated dosimetric calculation.

In some embodiments, the delivery system will organize the segments in such an order and configuration that the delivery can be accomplished in a least amount of time. This aspect of delivery time optimization may be accomplished during a delivery preparation phase. Further, this delivery time optimization may be accomplished by considering a single pass of 360 (or less) degrees of rotation, or multiple passes, as needed. In the instance of a single-pass mode, overlapping segments may be re-ordered and re-sized so that each segment can be delivered within the segment's own start angle and stop angle constraints. The segments may then be re-sorted so that they can be delivered on the expected gantry path.

It is noted that sequence of delivery segments described by a treatment planning system, sub-system, mechanism, or module need not be the same as the sequence of delivery segments reorganized by the intelligence of the delivery system.

In some embodiments, the Dynamic Strobe Modulated ARC Therapy delivery model disclosed herein may include modifying treatment table positions during the beam shaping phase of the beam delivery process to correct for variations in a patient's position.

In some embodiments, the systems may support clockwise and counterclockwise delivery, and the direction may be changed dynamically based upon where the gantry position is when the patient is positioned in the delivery system. For each segment, the gantry speed may be calculated such that all the parameters of the treatment plan can be achieved, taking into account the limitations of the system.

Those in the art will appreciate that various adaptations and modifications of the above-described embodiments can be configured without departing from the scope of the claims. Therefore, it is to be understood that the claims may be practiced other than as specifically described herein.

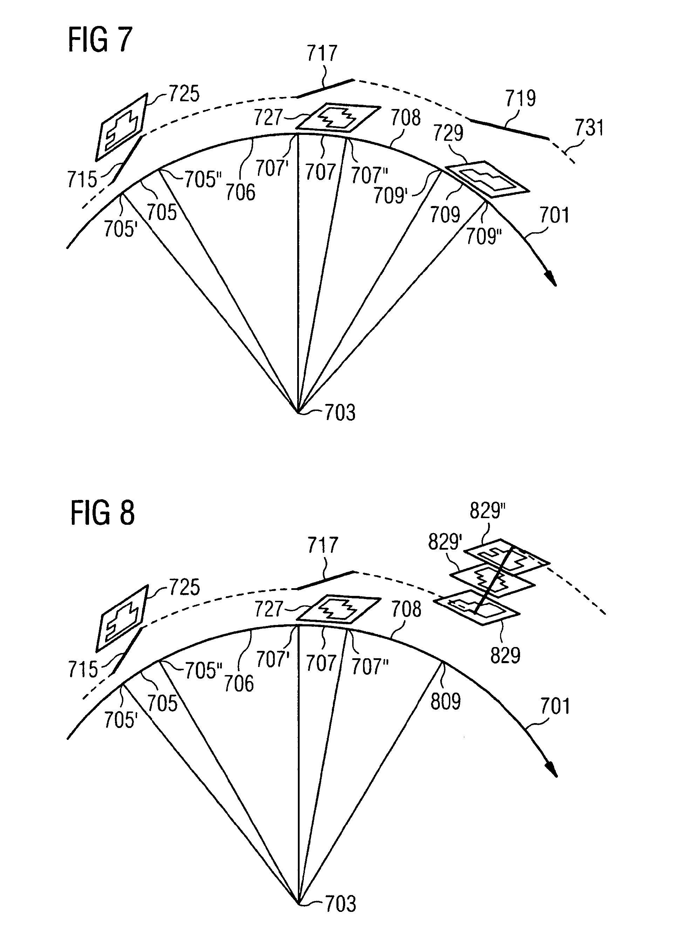

FIG. 7 provides an illustration of an embodiment of the invention. In this embodiment, a gantry moves along a circular arc 701 around an isocenter 703. The target volume (not shown) is positioned at the isocenter.

Three arclets 705, 707, 709 are shown that are distributed along the arc 701 of the gantry movement. Each arclet 705, 707, 709 is characterized by a start angle 705', 707', 709' and by a stop angle 705", 707", 709". As soon as the gantry reaches a start angle 705' of the first arclet 705 when moving along the arc 701, the radiation is turned on. The first radiation dose portion 715 is applied. Radiation is applied continuously as long as the gantry moves along the first arclet 705. The gantry motion is continuous along the first arclet 705. As soon as the gantry reaches the stop angle 705" of the arclet 705, radiation is turned off. During radiation delivery along the first arclet 705, beam parameters as beam energy, dose rate and beam shape 725 remain constant.

As soon as the radiation is turned off and the gantry moves along the gap 706 between the first arclet 705 and the second arclet 707, the beam energy, the dose rate and/or the beam shape 727 may be set to a new value. Also the gantry speed may be adjusted to a new value. Setting the new values takes place as the gantry keeps on moving along the gap 706 between the first arclet 705 and the second arclet 707. The gantry speed may be slowed down or even stopped in the gap 706 if the time for setting the new values would require it. However, the gantry can move as fast as possible to the next start angle 707' of the second arclet 707 to reduce overall time.

If desired, a verification step to verify the correct setting changes may be carried out before the gantry reaches the start angle 707' of the second arclet 707.

As soon as the gantry reaches the second arclet 707, the radiation is turned on again and the next radiation dose portion 717 is applied until the gantry reaches the stop angle 707" of the second arclet 707.

This application scheme continues for the following gaps 708 and arclets 709 until the last arclet has been reached and the last radiation dose portion (or burst of radiation) has been applied.

In this scheme, the dose applied to the target volume (dose curve 731) continuously increases at the arclets 705, 707, 709.

The gantry does not stop when moving through the arclets 705, 707, 709. The gantry may continuously move, eventually changing its speed, when moving through the gaps 706, 708 between the arclets 705, 707, 709.

FIG. 8 shows a different embodiment of the invention. The first and the second arclet 705, 707 correspond to the first and the second arclets as shown in FIG. 7. The third arclet 809, however, has a start angle that coincides with its stop angle. This arclet 809 has a point-like extension. In the gap 708 between the second arclet 707 and the third arclet 809, the beam parameters are set to the value required by the third arclet 809.

As soon as the gantry reaches the third arclet 809, the gantry movement stops. After stopping, a first radiation dose portion for the third arclet 809 is applied. After that, radiation is turned off and beam parameters (as different beam shapes 829, 829', 829", for example) are changed. As soon as the new parameter settings are implemented, the radiation is turned on again and a second radiation dose portion for the third arclet 809 is applied. This scheme may be repeated until the last radiation dose portion for the third arclet 809 is applied. Afterwards, the gantry resumes its rotation and moves until the next arclet is reached.

For point-like arclets as the third arclet 809, beam parameters may even change during the application of radiation. For example, settings of a beam shaping device may change as radiation passes through the beam shaping device.

The radiation may be applied to patients, for example to treat diseases as tumors. However, it is also possible to irradiate inanimate matter as phantoms or the like, for example for research or calibration purposes.

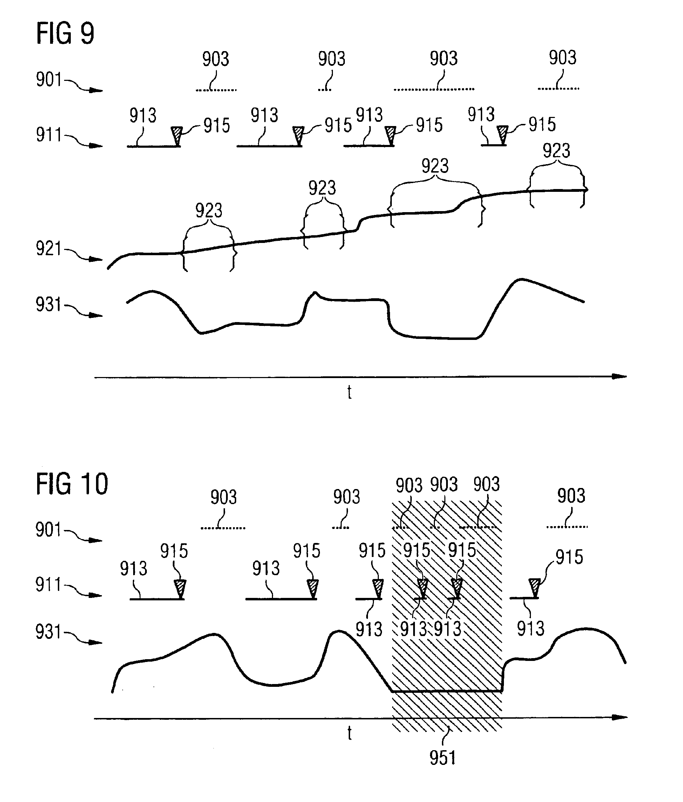

FIG. 9 shows an illustration of the dynamic process of radiation application according to the dynamic strobe scheme.

The upper part 901 of the diagram shows the periods the radiation is turned on (dotted line segments 903). Below the upper part a discrete parameter 911 is shown, e.g. the collimator setting. The plain line segments 913 indicate the time periods where the discrete parameter is being changed. These periods are arranged in the gaps between the periods the radiation is turned on. At the end of the time periods where the discrete parameter was changed a verification step 915 may be made to verify the correct setting. The behavior of the discrete parameter during the time periods relating to discrete parameter changes may be completely non-linear.

Below the time course of the discrete parameter 911, a continuous parameter 921 is shown, i.e. a parameter that changes during the periods where radiation is applied and during the gaps between those periods. The brackets 923 indicate predefined radiation application windows. Such windows correspond to predefined path sections that impose a constraint on actual path sections where radiation is applied. Actual path sections must fit within predefined path sections. The system may not allow application of radiation outside those windows.

Below the time course of the continuous parameter 921, another continuous parameter 931 is shown. This parameter may be modulated according to the speed of the dynamic component.

FIG. 10 shows an illustration of the dynamic process of radiation application according to the dynamic strobe scheme.

In this diagram, a static phase 951 is shown that interrupts movement of the dynamic component. The speed modulated parameter 931 stops to vary at the static phase 951. During the static phase 951, radiation may be turned on and off several times. Parameter settings of discrete parameters 911 may be changed between the bursts of radiation. After finishing radiation at the static phase 951, the dynamic component may resume movement and radiation may applied according to the scheme shown in FIG: 9.

## Claims

1. A radiotherapy system comprising:
a memory to store a radiation treatment plan for delivering at least a portion of a prescribed radiation dose to a target volume from different incidence directions to the target volume; and a control unit in communication with the memory and operable to control a radiotherapy device to cause:
delivering a portion of the prescribed radiation dose to the target volume by:
moving a dynamic component along a path in order to dynamically change the incidence direction of the radiation beam to the target volume, wherein a plurality of path sections are disposed along the path, each of the path sections having a start position and a stop position, and
delivering a portion of the prescribed radiation dose to the target volume at the path sections by applying radiation between the start position and the stop position of each path section, respectively,
**characterized in that** some of the path sections have a start position differing from the stop position and radiation is applied continuously as the dynamic component moves along these path sections.

2. The system of claim 1, wherein a gap is arranged between at least some of successive path sections, respectively.

3. The system of claim 1, wherein a system parameter setting, in particular a beam shape and/or a beam energy, is kept constant when radiation is applied between the start position and the stop position for at least some of the path sections.

4. The system of claim 1, wherein all path sections have a start position differing from the stop position and radiation is applied continuously as the dynamic component moves along these path sections.

5. The system of claim 1, wherein at least one of the path sections have a start position equal to the stop position.

6. The system of claim 5, wherein a plurality of successive radiation dose portions with different system parameter settings are applied at the path section having the start position equal to the stop position.

7. The system of claim 1, further comprising converting a fixed point along the path associated with each of the radiation dose portions to the path section having a start position and a stop position.

8. The system of claim 1, wherein lengths of path sections are determined under the constraint that the path section lengths are less than a predefined path section length.

9. The system of claim 1, wherein a radiation dose is distributed substantially equally between the start position and the stop position for at least some of the path sections.

10. The system of claim 1, wherein the path sections are determined such that the total length of the path sections is less than a given percentage value of a whole path length the dynamic component moves along, in particular less than 60% of the whole path length.

11. The system of claim 1, wherein the path sections, a movement of the dynamic component and/or a dose rate for the application of radiation are determined such that an overall time where radiation is applied at the path sections is less than a given percentage value of an overall travel time of the dynamic component moving along the whole path, in particular less than 60% of the overall travel time.

12. A non-therapeutic method comprising:
receiving a radiation treatment plan for delivering at least a portion of a prescribed radiation dose to a target volume of a phantom from different incidence directions to the target volume; and delivering a portion of the prescribed radiation dose to the target volume by:
moving a dynamic component along a path in order to dynamically change the incidence direction of the radiation to the target volume, wherein a plurality of path sections are disposed along the path, each of the path sections having a start position and a stop position, and
delivering a portion of the prescribed radiation dose to the target volume at the path sections by applying radiation between the start position and the stop position of each path section, respectively,
**characterized in that** at least some of the path sections have a start position differing from the stop position and radiation is applied continuously as the dynamic component moves along these path sections.

13. A computer-implemented method for radiotherapy planning comprising:
determining at least a portion of a prescribed radiation dose to a target volume of a phantom to be applied from different incidence directions to the target volume; and
determining a plurality of path sections, the path sections being distributed along a path that characterizes a movement of a dynamic component, wherein the dynamic component is adapted to change the incidence direction of the radiation to the target volume, and wherein each of the path sections has a start position and a stop position, and
determining a plurality of radiation dose portions that are assigned to the path sections, respectively, to be applied between the start position and the stop position of the path sections, such that the total overall dose that is to be applied corresponds to a desired dose distribution,
**characterized in that** at least some of the path sections have a start position differing from the stop position in order to apply radiation continuously as the dynamic component moves along these path sections.

14. A radiotherapy planning system comprising:
a computer system with an input device for inputting treatment planning information to the computer and for allowing interaction with a user and with an output device for outputting processed treatment planning information, the computer system further comprising a processor being configured to:
to determine at least a portion of a prescribed radiation dose to a target volume to be applied from different incidence directions to the target volume; and
to determine a plurality of path sections, the path sections being distributed along a path that characterizes a movement of a dynamic component, wherein the dynamic component is adapted to change the incidence direction of the radiation to the target volume, and wherein each of the path sections has a start position and a stop position, and
and to determine a plurality of radiation dose portions that are assigned to each path section, respectively, to be applied between the start position and the stop position of the path sections, such that the total overall dose that is to be applied corresponds to a desired dose distribution,
**characterized in that** at least some of the path sections have a start position differing from the stop position in order to apply radiation continuously as the dynamic component moves along these path sections.

15. A medium having processor-executable instructions stored thereon, to cause the radiotherapy system of claim 1 to perform the following steps:
receiving a radiation treatment plan for delivering at least a portion of a prescribed radiation dose to a target volume in a series of individual treatment beams from different incidence direction to the target volume; and
delivering a portion of the prescribed radiation dose to the target volume by:
moving a dynamic component along a path in order to dynamically change the incidence direction of the radiation beam to the target volume, wherein a plurality of path sections are disposed along the path, each of the path sections having a start position and a stop position, and
delivering a portion of the prescribed radiation dose to the target volume at the path sections by applying radiation between the start position and the stop position of each path section, respectively,
**characterized in that** at least some of the path sections have a start position differing from the stop position in order to apply radiation continuously as the dynamic component moves along these path sections.

## Patentansprüche

1. Strahlentherapiesystem, welches umfasst:
einen Speicher zum Speichern eines Strahlenbehandlungsplans zum Zuführen wenigstens eines Teils einer vorgeschriebenen Strahlendosis zu einem Zielvolumen aus unterschiedlichen Einfallsrichtungen zu dem Zielvolumen; und eine Steuereinheit, die mit dem Speicher in Verbindung steht und dafür ausgebildet ist, eine Strahlentherapievorrichtung zu steuern, um Folgendes zu bewirken:
Zuführen eines Teils der vorgeschriebenen Strahlendosis zu dem Zielvolumen durch:
Bewegen einer dynamischen Komponente entlang eines Weges, um die Einfallsrichtung des Strahlenbündels zu dem Zielvolumen dynamisch zu ändern, wobei eine Vielzahl von Wegabschnitten entlang des Weges angeordnet ist, wobei jeder der Wegabschnitte eine Startposition und eine Stoppposition aufweist;
Zuführen eines Teils der vorgeschriebenen Strahlendosis zu dem Zielvolumen an den Wegabschnitten, indem zwischen der Startposition und der Stoppposition jedes Wegabschnitts jeweils Strahlung appliziert wird,
**dadurch gekennzeichnet, dass** einige der Wegabschnitte eine Startposition aufweisen, die von der Stoppposition verschieden ist, und Strahlung kontinuierlich appliziert wird, während sich die dynamische Komponente entlang dieser Wegabschnitte bewegt.

2. System nach Anspruch 1, wobei zwischen wenigstens einigen der aufeinanderfolgenden Wegabschnitte jeweils eine Lücke angeordnet ist.

3. System nach Anspruch 1, wobei eine Systemparametereinstellung, insbesondere eine Strahlform und/oder eine Strahlenergie, für wenigstens einige der Wegabschnitte konstant gehalten wird, wenn Strahlung zwischen der Startposition und der Stoppposition appliziert wird.

4. System nach Anspruch 1, wobei alle Wegabschnitte eine Startposition aufweisen, die von der Stoppposition verschieden ist, und Strahlung kontinuierlich appliziert wird, während sich die dynamische Komponente entlang dieser Wegabschnitte bewegt.

5. System nach Anspruch 1, wobei wenigstens einer der Wegabschnitte eine Startposition aufweist, die gleich der Stoppposition ist.

6. System nach Anspruch 5, wobei eine Vielzahl von aufeinanderfolgenden Strahlendosisteilen mit unterschiedlichen Systemparametereinstellungen an dem Wegabschnitt appliziert wird, bei dem die Startposition gleich der Stoppposition ist.

7. System nach Anspruch 1, welches ferner das Umwandeln eines festen Punktes entlang des Weges, der jedem der Strahlendosisteile zugeordnet ist, in den Wegabschnitt, der eine Startposition und eine Stoppposition aufweist, umfasst.

8. System nach Anspruch 1, wobei Längen von Wegabschnitten unter der Nebenbedingung bestimmt werden, dass die Wegabschnittslängen kleiner als eine vordefinierte Wegabschnittslänge sind.

9. System nach Anspruch 1, wobei eine Strahlendosis für wenigstens einige der Wegabschnitte im Wesentlichen gleichmäßig zwischen der Startposition und der Stoppposition verteilt wird.

10. System nach Anspruch 1, wobei die Wegabschnitte derart bestimmt werden, dass die Gesamtlänge der Wegabschnitte kleiner als ein gegebener prozentualer Anteil einer ganzen Weglänge ist, entlang der sich die dynamische Komponente bewegt, insbesondere kleiner als 60 % der ganzen Weglänge.

11. System nach Anspruch 1, wobei die Wegabschnitte, eine Bewegung der dynamischen Komponente und/oder eine Dosisleistung für die Applikation von Strahlung derart bestimmt werden, dass eine Gesamtzeit, während der Strahlung an den Wegabschnitten appliziert wird, kleiner als ein gegebener prozentualer Anteil einer Gesamtbewegungszeit der dynamischen Komponente entlang des ganzen Weges ist, insbesondere kleiner als 60 % der Gesamtbewegungszeit.

12. Nichttherapeutisches Verfahren, welches umfasst:
Empfangen eines Strahlenbehandlungsplans zum Zuführen wenigstens eines Teils einer vorgeschriebenen Strahlendosis zu einem Zielvolumen eines Phantoms aus unterschiedlichen Einfallsrichtungen zu dem Zielvolumen; und Zuführen eines Teils der vorgeschriebenen Strahlendosis zu dem Zielvolumen durch:
Bewegen einer dynamischen Komponente entlang eines Weges, um die Einfallsrichtung der Strahlung zu dem Zielvolumen dynamisch zu ändern, wobei eine Vielzahl von Wegabschnitten entlang des Weges angeordnet ist, wobei jeder der Wegabschnitte eine Startposition und eine Stoppposition aufweist; und
Zuführen eines Teils der vorgeschriebenen Strahlendosis zu dem Zielvolumen an den Wegabschnitten, indem zwischen der Startposition und der Stoppposition jedes Wegabschnitts jeweils Strahlung appliziert wird,
**dadurch gekennzeichnet, dass** wenigstens einige der Wegabschnitte eine Startposition aufweisen, die von der Stoppposition verschieden ist, und Strahlung kontinuierlich appliziert wird, während sich die dynamische Komponente entlang dieser Wegabschnitte bewegt.

13. Computerimplementiertes Verfahren zur Strahlentherapieplanung, welches umfasst:
Bestimmen wenigstens eines Teils einer vorgeschriebenen Strahlendosis zu einem Zielvolumen eines Phantoms, die aus unterschiedlichen Einfallsrichtungen zu dem Zielvolumen zu applizieren ist; und
Bestimmen einer Vielzahl von Wegabschnitten, wobei die Wegabschnitte entlang eines Weges verteilt sind, welcher eine Bewegung einer dynamischen Komponente charakterisiert, wobei die dynamische Komponente dazu eingerichtet ist, die Einfallsrichtung der Strahlung zu dem Zielvolumen zu ändern, und wobei jeder der Wegabschnitte eine Startposition und eine Stoppposition aufweist; und
Bestimmen einer Vielzahl von Strahlendosisteilen, welche jeweils den Wegabschnitten zugeordnet sind, um zwischen der Startposition und der Stoppposition der Wegabschnitte appliziert zu werden, sodass die Gesamtdosis, welche zu applizieren ist, einer gewünschten Dosisverteilung entspricht,
**dadurch gekennzeichnet, dass** wenigstens einige der Wegabschnitte eine Startposition aufweisen, die von der Stoppposition verschieden ist, um kontinuierlich Strahlung zu applizieren, während sich die dynamische Komponente entlang dieser Wegabschnitte bewegt.

14. Strahlentherapieplanungssystem, welches umfasst:
ein Computersystem mit einer Eingabevorrichtung zum Eingeben von Behandlungsplanungsinformationen in den Computer und zum Ermöglichen einer Interaktion mit einem Benutzer und mit einer Ausgabevorrichtung zum Ausgeben von verarbeiteten Behandlungsplanungsinformationen, wobei das Computersystem ferner einen Prozessor umfasst, der dafür ausgebildet ist:
wenigstens einen Teil einer vorgeschriebenen Strahlendosis zu einem Zielvolumen zu bestimmen, die aus unterschiedlichen Einfallsrichtungen zu dem Zielvolumen zu applizieren ist; und
eine Vielzahl von Wegabschnitten zu bestimmen, wobei die Wegabschnitte entlang eines Weges verteilt sind, welcher eine Bewegung einer dynamischen Komponente charakterisiert, wobei die dynamische Komponente dazu eingerichtet ist, die Einfallsrichtung der Strahlung zu dem Zielvolumen zu ändern, und wobei jeder der Wegabschnitte eine Startposition und eine Stoppposition aufweist; und
eine Vielzahl von Strahlendosisteilen zu bestimmen, welche jeweils einem Wegabschnitt zugeordnet sind, um zwischen der Startposition und der Stoppposition der Wegabschnitte appliziert zu werden, sodass die Gesamtdosis, welche zu applizieren ist, einer gewünschten Dosisverteilung entspricht,
**dadurch gekennzeichnet, dass** wenigstens einige der Wegabschnitte eine Startposition aufweisen, die von der Stoppposition verschieden ist, um kontinuierlich Strahlung zu applizieren, während sich die dynamische Komponente entlang dieser Wegabschnitte bewegt.

15. Medium, auf dem von einem Prozessor ausführbare Anweisungen gespeichert sind, um zu bewirken, dass das Strahlentherapiesystem nach Anspruch 1 die folgenden Schritte ausführt:
Empfangen eines Strahlenbehandlungsplans zum Zuführen wenigstens eines Teils einer vorgeschriebenen Strahlendosis zu einem Zielvolumen in einer Reihe von individuellen Behandlungsbündeln aus unterschiedlichen Einfallsrichtungen zu dem Zielvolumen; und
Zuführen eines Teils der vorgeschriebenen Strahlendosis zu dem Zielvolumen durch:
Bewegen einer dynamischen Komponente entlang eines Weges, um die Einfallsrichtung des Strahlenbündels zu dem Zielvolumen dynamisch zu ändern, wobei eine Vielzahl von Wegabschnitten entlang des Weges angeordnet ist, wobei jeder der Wegabschnitte eine Startposition und eine Stoppposition aufweist; und
Zuführen eines Teils der vorgeschriebenen Strahlendosis zu dem Zielvolumen an den Wegabschnitten, indem zwischen der Startposition und der Stoppposition jedes Wegabschnitts jeweils Strahlung appliziert wird,
**dadurch gekennzeichnet, dass** wenigstens einige der Wegabschnitte eine Startposition aufweisen, die von der Stoppposition verschieden ist, um Strahlung kontinuierlich zu applizieren, während sich die dynamische Komponente entlang dieser Wegabschnitte bewegt.

## Revendications

1. Système de radiothérapie comprenant :
une mémoire pour enregistrer un plan de traitement par radiothérapie pour administrer au moins une portion d'une dose de rayonnement prescrite à un volume cible depuis différentes directions d'incidence vers le volume cible ;
et une unité de commande en communication avec la mémoire et pouvant fonctionner pour commander un appareil de radiothérapie pour obtenir :
l'administration d'une portion de la dose de rayonnement prescrite au volume cible par :
déplacement d'un composant dynamique le long d'un trajet afin de modifier dynamiquement la direction d'incidence du faisceau de rayonnement vers le volume cible, une pluralité de sections de trajet étant situées le long du trajet, chacune des sections de trajet ayant une position de départ et une position d'arrêt, et
administration d'une portion de la dose de rayonnement prescrite au volume cible au niveau des sections de trajet par application d'un rayonnement entre la position de départ et la position d'arrêt de chaque section de trajet, respectivement,
**caractérisé en ce que** certaines des sections de trajet ont une position de départ différente de la position d'arrêt et le rayonnement est appliqué de manière continue à mesure que le composant dynamique se déplace le long de ces sections de trajet.

2. Système selon la revendication 1, dans lequel un intervalle est ménagé entre au moins certaines des sections de trajet successives, respectivement.

3. Système selon la revendication 1, dans lequel un réglage des paramètres du système, notamment une forme d'un faisceau et/ou une énergie d'un faisceau, est maintenu constant pendant l'application du rayonnement entre la position de départ et la position d'arrêt pour au moins certaines des sections de trajet.

4. Système selon la revendication 1, dans lequel toutes les sections de trajet ont une position de départ différente de la position d'arrêt et le rayonnement est appliqué de manière continue à mesure que le composant dynamique se déplace le long de ces sections de trajet.

5. Système selon la revendication 1, dans lequel au moins une des sections de trajet a une position de départ identique à la position d'arrêt.

6. Système selon la revendication 5, dans lequel une pluralité de portions de dose de rayonnement successives avec différents réglages des paramètres du système sont appliquées au niveau de la section de trajet dont la position de départ est identique à la position d'arrêt.

7. Système selon la revendication 1, comprenant, en outre, la conversion d'un point fixe le long du trajet, associé à chacune des portions de dose de rayonnement, en la section de trajet ayant une position de départ et une position d'arrêt.

8. Système selon la revendication 1, dans lequel des longueurs de sections de trajet sont déterminées, avec pour contrainte que les longueurs de section de trajet sont inférieures à une longueur de section de trajet prédéfinie.

9. Système selon la revendication 1, dans lequel une dose de rayonnement est distribuée de manière sensiblement égale entre la position de départ et la position d'arrêt pour au moins certaines des sections de trajet.

10. Système selon la revendication 1, dans lequel les sections de trajet sont déterminées de manière à ce que la longueur totale des sections de trajet soit inférieure à une valeur correspondant à un pourcentage donné de la longueur d'un trajet entier le long duquel le composant dynamique se déplace, notamment inférieure à 60% de la longueur du trajet entier.

11. Système selon la revendication 1, dans lequel les sections de trajet, un déplacement du composant dynamique et/ou un débit de dose pour l'application du rayonnement sont déterminés de manière à ce qu'une durée globale d'application du rayonnement au niveau des sections de trajet soit inférieure à une valeur correspondant à un pourcentage donné d'un temps de déplacement total du composant dynamique se déplaçant le long du trajet entier, notamment inférieure à 60% du temps de déplacement total.

12. Procédé non thérapeutique comprenant :
la réception d'un plan de traitement par radiothérapie pour administrer au moins une portion d'une dose de rayonnement prescrite à un volume cible d'un fantôme depuis différentes directions d'incidence vers le volume cible ; et l'administration d'une portion de la dose de rayonnement prescrite au volume cible par :
déplacement d'un composant dynamique le long d'un trajet afin de modifier dynamiquement la direction d'incidence du rayonnement vers le volume cible, une pluralité de sections de trajet étant situées le long du trajet, chacune des sections de trajet ayant une position de départ et une position d'arrêt, et
administration d'une portion de la dose de rayonnement prescrite au volume cible au niveau des sections de trajet par application d'un rayonnement entre la position de départ et la position d'arrêt de chaque section de trajet, respectivement,
**caractérisé en ce qu'**au moins certaines des sections de trajet ont une position de départ différente de la position d'arrêt et le rayonnement est appliqué de manière continue à mesure que le composant dynamique se déplace le long de ces sections de trajet.

13. Procédé de planification de radiothérapie mis en oeuvre par ordinateur, comprenant :
la détermination d'au moins une portion d'une dose de rayonnement prescrite pour un volume cible d'un fantôme à être appliquée au volume cible depuis différentes directions d'incidence vers le volume cible ; et
la détermination d'une pluralité de sections de trajet, les sections de trajet étant réparties le long d'un trajet qui caractérise un déplacement d'un composant dynamique, le composant dynamique étant adapté pour modifier la direction d'incidence du rayonnement vers le volume cible et chacune des sections de trajet ayant une position de départ et une position d'arrêt, et
la détermination d'une pluralité de portions de dose de rayonnement qui sont assignées aux sections de trajet, respectivement, pour être appliquées entre la position de départ et la position d'arrêt des sections de trajet de manière à ce que la dose globale totale à appliquer corresponde à une distribution de dose souhaitée,
**caractérisé en ce qu'**au moins certaines des sections de trajet ont une position de départ différente de la position d'arrêt afin d'appliquer un rayonnement de manière continue à mesure que le composant dynamique se déplace le long de ces sections de trajet.

14. Système de planification de radiothérapie, comprenant :
un système informatique pourvu d'un dispositif d'entrée pour entrer des informations de planification de traitement dans l'ordinateur et pour permettre l'interaction avec un utilisateur, et d'un dispositif de sortie pour livrer en sortie des informations de planification de traitement traitées, le système informatique comprenant, en outre, un processeur configuré :
pour déterminer au moins une portion d'une dose de rayonnement prescrite pour un volume cible à être appliquée depuis différentes directions d'incidence vers le volume cible ; et
pour déterminer une pluralité de sections de trajet, les sections de trajet étant réparties le long d'un trajet qui caractérise un déplacement d'un composant dynamique, le composant dynamique étant adapté pour modifier la direction d'incidence du rayonnement vers le volume cible, et chacune des sections de trajet ayant une position de départ et une position d'arrêt, et
pour déterminer une pluralité de portions de dose de rayonnement qui sont assignées à chaque section de trajet, respectivement, pour être appliquées entre la position de départ et la position d'arrêt des sections de trajet, de manière à ce que la dose globale totale à être appliquée corresponde à une distribution de dose souhaitée,
**caractérisé en ce qu'**au moins certaines des sections de trajet ont une position de départ différente de la position d'arrêt afin d'appliquer un rayonnement de manière continue à mesure que le composant dynamique se déplace le long de ces sections de trajet.

15. Support sur lequel sont enregistrées des instructions pouvant être exécutées par un processeur pour causer l'exécution par le système de radiothérapie selon la revendication 1 des étapes suivantes :
réception d'un plan de traitement par radiothérapie pour administrer au moins une portion d'une dose de rayonnement prescrite à un volume cible en une série de faisceaux de traitement individuels depuis différentes directions d'incidence vers le volume cible ; et
administration d'une portion de la dose de rayonnement prescrite au volume cible par :
déplacement d'un composant dynamique le long d'un trajet afin de modifier dynamiquement la direction d'incidence du faisceau de rayonnement vers le volume cible, une pluralité de sections de trajet étant situées le long du trajet, chacune des sections de trajet ayant une position de départ et une position d'arrêt, et
administration d'une portion de la dose de rayonnement prescrite au volume cible au niveau des sections de trajet en appliquant un rayonnement entre la position de départ et la position d'arrêt de chaque section de trajet, respectivement,
**caractérisé en ce qu'**au moins certaines des sections de trajet ont une position de départ différente de la position d'arrêt afin d'appliquer un rayonnement de manière continue à mesure que le composant dynamique se déplace le long de ces sections de trajet.
